(19) Europäisches Patentamt
European Patent Office
Office européen des brevets

(11) Veröffentlichungsnummer: **0 161 588**
**B1**

(12) **EUROPÄISCHE PATENTSCHRIFT**

(45) Veröffentlichungstag der Patentschrift: **16.08.90**

(21) Anmeldenummer: **85105391.8**

(22) Anmeldetag: **03.05.85**

(51) Int. Cl.$^5$: **C 07 K 5/00,** C 07 K 5/02, C 07 K 7/00, C 07 K 7/02, C 07 C 229/26, C 07 C 271/12

(54) **Diaminosäurederivate.**

(30) Priorität: **18.05.84 DE 3418491**

(43) Veröffentlichungstag der Anmeldung: **21.11.85 Patentblatt 85/47**

(45) Bekanntmachung des Hinweises auf die Patenterteilung: **16.08.90 Patentblatt 90/33**

(84) Benannte Vertragsstaaten: **AT BE CH DE FR GB IT LI NL SE**

(56) Entgegenhaltungen:
**EP-A-0 081 783**
**EP-A-0 118 223**
**EP-A-0 155 809**
**EP-A-0 156 322**
**WO-A-84/03044**

**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **Raddatz, Peter, Dr.**
**Grafenstrasse 14**
**D-6100 Darmstadt (DE)**
Erfinder: **Schmitges, Claus, Dr.**
**Karolingerstrasse 5**
**D-6114 Gross-Umstadt (DE)**

**EP 0 161 588 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Beschreibung

Die Erfindung betrifft neue Diaminosäurederivate der Formel I

$$X—Z—NH—CH(CH_2R^1)—CH(NH_2)—CH_2—CO—E—G—Y \qquad I$$

worin

- X: H, $R^2—O—C_nH_{2n}—CO—$, $R^2—C_nH_{2n}—O—CO—$, $R^2—C_nH_{2n}—CO—$, $R^2—SO_2—$, $(R^2—C_nH_{2n})—L(R^2—C_rH_{2r})—C_tH_{2t}—CO—$, $H—(NHCH_2CH_2)_n—NH—CH_2CO—$ oder 9-Fluorenyl—$C_nH_{2n}—O—CO—$,
- Z: 0 bis 4 peptidartig miteinander verbundene Aminosäurereste ausgewählt aus der Gruppe bestehend aus Abu, Ada, Ala, Arg, Dab, Gly, His, Ile, Leu, tert.-Leu, Lys, Met, Nbg, Nle, N-Me-His, N-Me-Phe, Orn, Phe, Pro, Ser, Thr, Tic, Trp, Tyr und Val,
- $R^1$: H, A, Cycloalkyl mit 3—7 C-Atomen, Ar oder $C_pH_{2p}$-W,
- E: abwesend, Ala, Gly, Ile, Leu, tert.-Leu, Met, Ser, Thr oder Val,
- G: $—NH—CH(CH_2R^1)—CH(NH_2)—CH_2—CO—$,
- Y: $—O—C_mH_{2m}—R^3$, $—NH—C_mH_{2m}—R^3$, $—NH—C_mH_{2m-1}(R^3)_2$ oder $NA_2$,
- $R^2$: A, Cycloalkyl mit 3—7 C-Atomen, Benzyl oder Ar,
- L: CH oder N,
- $R^3$: H, A, Cycloalkyl mit 3—7 C-Atomen, Ar, Pyridyl, Imidazolyl, Piperidyl, N-Benzyl-piperidyl oder Piperazinyl,
- W: OH, $NH_2$, OA, NHA oder $NA_2$,
- A: Alkyl mit 1—6 C-Atomen,
- Ar: unsubstituiertes oder ein- oder mehrfach durch A, AO, F, Cl, Br, J, $CF_3$ und/oder $NH_2$ substituiertes Phenyl oder unsubstituiertes Naphthyl und

m, n, p, r und t jeweils 0, 1, 2, 3, 4 oder 5 bedeuten,
sowie deren Salze.

Ähnliche Verbindungen sind aus der EP—A—77028, aus der EP—A—0 118 223, aus der EP—A—0 156 322 und aus der EP—A—0 155 809 bekannt; jedoch enthalten sie alle höchstens eine "Amino-statin"-Einheit.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasma-renins. Diese Wirkung kann z.B. nach der Methode von F. Fyhrquist et. al., Clin. Chem. *22*, 250—256 (1976), nachgewiesen werden. Bemerkenswert ist, daß diese Verbindungen sehr spezifische Hemmer des Renins sind; für die Hemmung anderer Aspartylproteinasen (z.B. Pepsin und Kathepsin D) sind wesentlich höhere Konzentrationen dieser Verbindungen notwendig.

Die Verbindungen können als Arzneimittelwirkstoffe in der Human- und Veterinärmedizin eingesetzt werden, insbesondere zur Propylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor- und nachstehend aufgeführten Abkürzungen von Aminosäureresten stehen für die Reste —NH—CHR—CO— (worin R die für jede Aminosäure bekannte spezifische Bedeutung hat) folgender Aminosäuren:

| | |
|---|---|
| Abu | 2-Aminobuttersäure |
| Ada | Adamantylalanin |
| Ala | Alanin |
| Arg | Arginin |
| Dab | 2,4-Diaminobuttersäure |
| Gly | Glycin |
| His | Histidin |
| Ile | Isoleucin |
| Leu | Leucin |
| tert.-Leu | tert.-Leucin |
| Lys | Lysin |
| Met | Methionin |
| Nbg | (2-Norbornyl)-glycin |
| Nle | Norleucin |
| N-Me-His | N-Methyl-histidin |
| N-Me-Phe | N-Methyl-phenylalanin |
| Orn | Ornithin |
| Phe | Phenylalanin |
| Pro | Prolin |
| Ser | Serin |
| Thr | Threonin |
| Tic | Tetrahydroisochinolin-1-carbonsäure |
| Trp | Tryptophan |
| Tyr | Tyrosin |
| Val | Valin. |

Ferner bedeutet nachstehend:

| | |
|---|---|
| BOC | tert.-Butoxycarbonyl |
| CBZ | Benzyloxycarbonyl |
| DNP | 2,4-Dinitrophenyl |
| FMOC | 9-Fluorenylmethoxycarbonyl |
| imi-DNP | 2,4-Dinitrophenyl in 1-Stellung des Imidazolrings |
| OMe | Methylester |
| POA | Phenoxyacetyl |
| DCCI | Dicyclohexylcarbodiimid |
| HOBt | 1-Hydroxybenzotriazol. |

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Die beiden in I vorhandenen Reste $R^1$ können gleich oder voneinander verschieden sein.

Besonders bevorzugte Verbindungen der Formel I sind solche, die von 3,4-Diamino-6-methylheptansäure ("DAMH") und von 3,4-Diamino-5-phenylpentansäure ("DAPP"), ferner von 3,4-Diamino-5-cyclohexylpentansäure ("DACP") abgeleitet sind.

Die Verbindungen der Formel sowie deren Derivate besitzen in der Gruppe $—NH—CH(CH_2R^1)—CH(NH_2)—CH_2—CO—$ mindestens zwei chirale Zentren. Sie können daher in verschiedenen—optisch-inaktiven oder optisch-aktiven—Formen vorkommen. Die Formel umschließt alle diese enantiomeren Formen. Die 3S,4S-Diamino-Enantiomeren sind bevorzugt. Wenn nichts anderes angegeben ist, beziehen sich die Abkürzungen DAMH, DAPP und DACP immer auf diese 3S,4S-Formen.

In den vorstehenden Formel hat A 1—6, vorzugsweise 1,2,3 oder 4 C-Atome. A bedeutet vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl.

Cycloalkyl bedeutet vorzugsweise Cyclopropyl, Cyclobutyl, Cyclopentyl, Cyclohexyl oder Cycloheptyl, aber auch z.B. 1-, 2- oder 3-Methylcyclopentyl, 1-, 2-, 3- oder 4-Methylcyclohexyl.

Ar bedeutet vorzugsweise Phenyl, ferner o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m-oder p-Jodphenyl, o-, m- oder p-Trifluormethylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, 1- oder 2-Naphthyl.

$R^1$ bedeutet vorzugsweise H; A, insbesondere Methyl, Ethyl, Propyl, Isopropyl, Isobutyl oder sek.-Butyl; Cyclohexyl; Phenyl; p-Chlorphenyl; OH; Hydroxyalkyl wie Hydroxymethyl; $NH_2$; Aminoalkyl wie Aminomethyl, 1- oder 2-Aminoethyl, 1-, 2- oder 3-Aminopropyl; Alkoxy wie Methoxy, Ethoxy, Propoxy, Isopropoxy oder Butoxy; Alkoxyalkyl wie Methoxymethyl, Ethoxymethyl, Propoxymethyl, Isopropoxymethyl, 1- oder 2-Methoxyethyl, 1- oder 2-Ethoxyethyl, 1- oder 2-Propoxyethyl, 1- oder 2-Isopropoxyethyl, 1-, 2- oder 3-Methoxypropyl; Alkylamino wie Methylamino, Ethylamino, Propylamino, Isopropylamino; Alkylaminoalkyl wie Methylaminomethyl, Ethylaminomethyl, Propylaminomethyl, Isopropylaminomethyl, 1- oder 2-Methylaminoethyl, 1- oder 2-Ethylaminoethyl, 1- oder 2-Propylaminoethyl, 1- oder 2-Isopropylaminoethyl, 1-, 2- oder 3-Methylaminopropyl; Dialkylamino wie Dimethylamino, Methylethylamino, Diethylamino; Dialkylaminoalkyl wie Dimethylamino, Methylethylamino, Diethylamino; Dialkylaminoalkyl wie Dimethylaminomethyl, Methylethylaminomethyl, Diethylaminomethyl, 1- oder 2-Dimethylaminoethyl, 1- oder 2-Methylethylaminoethyl, 1- oder 2-Diethylaminoethyl, 1-, 2- oder 3-Dimethylaminopropyl. Besonders bevorzugte Reste $R^1$ sind Isopropyl und Phenyl, in zweiter Linie H, Ethyl, OH, 2-Aminoethyl und 3-Aminopropyl.

$R^2$ bedeutet vorzugsweise A, insbesondere Methyl, Ethyl, Propyl, Isopropyl, Butyl, Isobutyl oder tert.-Butyl; Cyclopropyl, Cyclopentyl, Cyclohexyl, Benzyl oder Phenyl.

$R^3$ bedeutet vorzugsweise H, A, insbesondere Methyl, Cyclohexyl, Phenyl, o-, m- oder p-Fluorphenyl, o-, m- oder p-Chlorphenyl, o-, m- oder p-Bromphenyl, o-, m- oder p-Aminophenyl, 2-, 3- oder (insbesondere) 4-Pyridyl, 1-, 2-, (insbesondere) 4- oder 5-Imidazolyl, 1-, 2-, 3- oder (insbesondere) 4-Piperidyl, N-Benzyl-2-, -3- oder (insbesondere) -4-piperidyl, 1-, 2- oder 3-Piperazinyl.

X bedeutet vorzugsweise H, POA, Alkoxycarbonyl wie BOC, CBZ, Alkanoyl wie Acetyl, Propionyl, Butyryl oder Isobutyryl, Cycloalkylcarbonyl wie Cyclopentylcarbonyl oder Cyclohexylcarbonyl, Aroyl wie Benzoyl, Arylalkanoyl wie Phenylacetyl, 2- oder 3-Phenylpropionyl, 2- oder 3-o-, -m- oder -p-Fluorphenylpropionyl, 2- oder 3-o-, -m- oder -p-Chlorphenylpropionyl, Cycloalkylalkanoyl wie Cyclohexylacetyl, 2- oder 3-Cyclohexylpropionyl. Besonders bevorzugte Reste X sind H, BOC und CBZ.

Z bedeutet O (=Valenz) oder 1, vorzugsweise 2, 3 oder 4 peptidartig miteinander verbundene Aminosäurereste, insbesondere die Gruppen His, Phe-His, Pro-Phe-His oder His-Pro-Phe-His, ferner bevorzugt die Gruppen Abu-His, Ada-His, Ala-His, Ala-Phe, Arg-His, Dab-His, Gly-His, His-His Ile-His, Leu-His, tert.-Leu-His, Lys-His, Met-His, Nbg-His, Nle-His, (N-Me-His)-His, (N-Me-Phe)-His, Orn-His, Phe-Abu, Phe-Ada, Phe-Ala, Phe-Arg, Phe-Dab, Phe-Gly, Phe-Ile, Phe-Leu, Phe-tert.-Leu, Phe-Lys, Phe-Met, Phe-Nbg, Phe-Nle, Phe-(N-Me-His), Phe-(N-Me-Phe), Phe-Orn, Phe-Phe, Phe-Pro, Phe-Ser, Phe-Thr, Phe-Tic, Phe-Trp, Phe-Tyr, Phe-Val, Pro-His, Ser-His, Thr-His, Tic-His, Trp-His, Tyr-His, Val-His, Pro-Ala-His, Pro-Ala-Phe, Pro-Phe-Ala, Pro-Phe-Phe, His-Pro-Ala-His, His-Pro-Ala-Phe, His-Pro-Phe-Ala, His-Pro-Phe-Phe, weiterhin Pro-Abu-His, Pro-Ada-His, Pro-Arg-His, Pro-Dab-His, Pro-Gly-His, Pro-His-His, Pro-Ile-His, Pro-Leu-His, Pro-tert.-Leu-His, Pro-Lys-His, Pro-Met-His, Pro-Nbg-His, Pro-Nle-His, Pro-(N-Me-His)-His, Pro-(N-Me-Phe)-His, Pro-Orn-His, Pro-Phe-Abu, Pro-Phe-Ada, Pro-Phe-Arg, Pro-Phe-Dab, Pro-Phe-Gly, Pro-Phe-Ile, Pro-Phe-Leu, Pro-Phe-tert.-Leu, Pro-Phe-Lys, Pro-Phe-Met, Pro-Phe-Nbg, Pro-Phe-Nle, Pro-Phe-(N-Me-His), Pro-Phe-(N-Me-Phe), Pro-Phe-Orn, Pro-Phe-Pro, Pro-Phe-Ser, Pro-Phe-Thr, Pro-Phe-Tic, Pro-Phe-Trp, Pro-Phe-Tyr, Pro-Phe-Val, Pro-Pro-His, Pro-Ser-His, Pro-Thr-His, Pro-Tic-His, Pro-Trp-His, Pro-Tyr-His, Pro-Val-His, His-Pro-Abu-His, His-Pro-Ada-His, His-Pro-Arg-His, His-Pro-Dab-His, His-Pro-Gly-His, His-Pro-His-His, His-Pro-Ile-His, His-Pro-Leu-His, His-Pro-tert.-Leu-His, His-Pro-Lys-His, His-Pro-Met-His, His-Pro-Nbg-His, His-Pro-Nle-His, His-Pro-(N-Me-His)-His, His-Pro-(N-Me-Phe)-His, His-Pro-Orn-His, His-Pro-Phe-Abu, His-Pro-Phe-Ada, His-Pro-Phe-Arg, His-Pro-Phe-Dab, His-Pro-Phe-Gly, His-Pro-Phe-Ile, His-Pro-Phe-Leu, His-Pro-Phe-tert.-Leu, His-Pro-Phe-Lys, His-Pro-Phe-Met, His-Pro-Phe-Nbg, His-Pro-Phe-Nle, His-Pro-Phe-(N-Me-His), His-Pro-Phe-(N-Me-Phe), His-Pro-Phe-Orn, His-Pro-Phe-Pro, His-Pro-Phe-Ser, His-Pro-Phe-Thr, His-Pro-Phe-Tic, His-Pro-Phe-Trp, His-Pro-Phe-Tyr, His-Pro-Phe-Val, His-Pro-Pro-His, His-Pro-Ser-His, His-Pro-Thr-His, His-Pro-Tic-His, His-Pro-Trp-His, His-Pro-Tyr-His, His-Pro-Val-His.

E ist vorzugsweise abwesend oder bedeutet vorzugsweise Ile, ferner vorzugsweise Leu, weiterhin Ala, Gly, Met, Ser, Thr oder Val.

Die Gruppe —E—G— bedeutet vorzugweise Ile—NH—$CH(CH_2R^1)$—($CHNH_2$)—$CH_2$—CO—.

Y ist vorzugsweise $OR^3$, insbesondere OA, oder —NH—$C_mH_{2m}$—$R^3$, wobei die Gruppe $C_mH_{2m}$ bevorzugt geradkettiges Alkylen mit 1—5 C-Atomen bedeutet, insbesondere —$CH_2$—, —$CH_2CH_2$—, —$(CH_2)_3$—, ferner auch —$(CH_2)_4$— oder —$(CH_2)_5$)—, aber auch z.B. —$CH(CH_3)$—, —$CH(CH_3)$—$CH_2$— oder —$CH_2$—$CH(CH_3)$—. Eine bevorzugte Bedeutung der Gruppe —NH—$C_mH_{2m}$—$R^3$ ist auch $NH_2$. Die Gruppe —NH—$C_mH_{2m-1}(R^3)_2$ ist bevorzugt —NH—$CH_2CH(Ar)_2$.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden teilformeln Ia bis If ausgedrückt werden, die der Formel I entsprechen, worin jedoch

in Ia (a) Z 3 oder 4 peptidartig miteinander verbundene Aminosäurereste ausgewählt aus der Gruppe bestehend aus Abu, Ada, Ala, Arg, Dab, Gly, His, Ile, Leu, tert.-Leu, Lys, Met, Nbg, Nle, N-Me-His, N-Me-Phe, Orn, Phe, Pro, Ser, Thr, Tic, Trp, Tyr und Val bedeutet und/oder

(b) $R^1$ $C_pH_{2p}$—W und
W OA, NHA oder $NA_2$ bedeuten und/oder
(c) Y —O—$C_mH_{2m}$—$R^3$ oder —NH—$C_mH_{2m}$—$R^3$,
m 2, 3, 4 oder 5 und
$R^3$ Pyridyl, Imidazolyl, Piperidyl, N-Benzylpyridyl oder Piperazinyl bedeutet;

in Ib Z 3 oder 4 peptidartig miteinander verbundene Aminosäurereste ausgewählt aus der Gruppe bestehend aus Abu, Ada, Ala, Arg, Dab, Gly, His, Ile, Leu, tert.-Leu, Lys, Met, Nbg, Nle, N-Me-His, N-Me-Phe, Orn, Phe, Pro, Ser, Thr, Tic, Trp, Tyr und Val bedeutet;

in Ic Z 3 oder 4 peptidartig miteinander verbundene Aminosäurereste ausgewählt aus der Gruppe bestehend aus His, Phe und Pro bedeutet;

in Id Z Pro-Phe-His oder His-Pro-Phe-His bedeutet;

in Ie G DAMH, DAPP oder DACP bedeutet;

in If G DAMH bedeutet.

Gegenstand der Erfindung ist ferner ein Verfahren zur Herstellung eines Diaminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man ein Aminoketosäurederivat der Formel II

$$X—Z—NH—CH(CH_2R^1)—CO—CH_2—CO—E—G—Y \quad II$$

worin

$R^1$, E, G, X, Y und Z die bei Formel I angegebene Bedeutung haben reduktiv aminiert und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine Verbindung der Formel I durch Behandeln mit einer Säure oder Base in eines ihrer Salze überführt.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart; ferner EP—A—45665, EP—A—77028, EP—A—77029, EP—A—81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isolierte, sondern sofort weiter zu den Verbindungen der Formel I umsetzt.

Vorzugsweise werden die Verbindungen der Formel I erhalten, indem man sie aus ihreren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino- und/oder Hydroxygruppen entsprechende geschützte Amino- und/oder Hydroxygruppen enthalten, vorzugsweise solche, die an stelle eines H-Atoms, das mit einem N-Atom verbunden ist, eine Aminoschutzgruppe tragen, insbesondere diejenigen der Formel III

$$X—Z—CH(CH_2R^1)—CH(NHQ)—CH_2—CO—E—G—Y \quad III$$

worin

Q eine Aminoschutzgruppe bedeutet.

Ferner sind Ausgangsstoffe bevorzugt, die an Stelle des H-Atoms einer Hydroxygruppe eine Hydroxyschutzgruppe tragen.

Es können auch mehrere—gleiche oder verschiedene—geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acylgruppen, ferner unsubstituierte oder substituierte Aryl-(z.B. 2,4-Dinitrophenyl) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1—20, insbesondere 1—8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl;

Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Acylgruppen sind CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1—20, insbesondere 1—10 C-Atomen. Biespiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind.

Das In-Freiheit-Setzen der Verbindungen der Formel I aus ihren funktionellen Derivaten gelingt—je nach der benutzten Schutzgruppe—z.B. mit starken Säuren, zweckmäßig mit Trifluoressigsäure oder Perchlorsäure aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Methylenchlorid, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z.B. bevorzugt mit 40 %iger Trifluoressigsäure in Methylenchlorid oder mit etwa 3 bis 5 n HCl in Dioxan bei 15—30° abgespalten werden, die FMOC-Gruppe mit einer etwa 5- bis 20 %igen Lösung von Dimethylamin, Diethylamin oder Piperidin in DMF bei 15—30°. Eine Abspaltung der DNP-Gruppe gelingt z.B. auch mit einer etwa 3- bis 10 %igen Lösung von 2-Mercaptoethanol in DMF/Wasser bei 15—30°.

Hydrogenolytisch entfernbare Schutzgruppen (z.B. CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20—30° und 1—10 bar durchgeführt. Eine Hydrogenolyse der CBZ-Gruppe gelingt z.B. gut an 5-bis 10 %igem Pd-C in Methanol bei 20—30°.

Die Verbindungen der Formel I können auch durch reduktive Aminierung von Aminoketosäurederivaten der Formel II hergestellt werden.

Diese sind z.B. aus Aminosäuren der Formel X—Z—NH—CH($CH_2R^1$)—COOH durch Umsetzung mit Carbonyldiimidazol in die entsprechenden Imidazolide und anschließende Reaktion mit Malonsäurederivaten der Formel HOOC—$CH_2$—CO—E—G—Y bzw. deren Salzen erhältlich.

Man kann ein- oder mehrstufig reduktiv aminieren. So kann man die Verbindung II mit Ammoniumsalzen, z.B. Ammoniumacetat, und $NaCNBH_3$ behandeln, vorzugsweise in einem inerten Lösungsmittel, z.B. einem Alkohol wie Methanol bei Temperaturen zwischen etwa 0 und 50°, insbesondere zwischen 15 und 30°.

Weiterhin ist es möglich, das Keton II zunächst mit Hydroxylamin in üblicher Weise in das Oxim zu überführen und dieses, z.B. durch katalytische Hydrierung an Raney-Nickel, zum Amin zu reduzieren.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X=H) umgewandelt werden, zweckmäßig durch Hydrogenolyse, falls X=CBZ ist, sonst durch selektive Solvolyse. Falls X=BOC ist, kann man z.B. mit HCl in Dioxan bei Raumtemperatur die BOC-Gruppe abspalten.

Weiterhin ist es z.B. möglich, einen Ester der Formel I (Y=—O—$C_mH_{2m}R^3$) zur entsprechenden Säure der Formel I (Y=OH) zu verseifen, z.B. mit wässerig-dioxanischer Natronlauge bei Raumtemperatur.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische,

araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Eine Säure der Formel I kann durch Umsetzung mit einer Base in eines ihrer physiologisch unbedenklichen Metall-bzw. Ammoniumsalze übergeführt werden. Als Salze kommen insbesondere die Natrium-, Kalium-, Magnesium-, Calcium- und Ammoniumsalze in Betracht, ferner substituierte Ammoniumsalze, z.B. die Dimethyl-, Diethyl- oder Diisopropylammonium-, Monoethanol-, Diethanol- und Triethanol- ammonium-, Cyclohexylammonium-, Dicyclohexylammonium- und Dibenzylethylendiammoniumsalze, weiterhin z.B. Salze mit N-Methyl-D-Glucamin oder mit basischen Aminosäuren wie Arginin der Lysin.

Die neuen Verbindungen der Formel I und ihre physiologisch unbedenklichen Salze können zur Herstellung pharmazeutischer Präparate verwendet werden, indem man sie zusammen mit mindestens einen Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin. Zur rektalen Anwendung dienen Supositiorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlor-kohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthälten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP—A—77028 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Man löst 1 g 3S-CBZ-amino-4S-(POA-His-amino)-5-phenylpentanoyl-Ile-Phe-OMe [erhältlich durch Reaktion von BOC-DAPP-OMe mit Benzyloxycarbonylchlorid zu 3S-CBZ-amino-4S-BOC-amino-5-phenylpentansäuremethylester (F. 111—112°), Verseifung zu 3S-CBZ-amino-4S-BOC-amino-5-phenylpentansäure (F. 126—127°), Umsetzung mit H-Ile-Phe-OMe zu 3S-CBZ-amino-4S-BOC-amino-5-phenylpentanoyl-Ile-Phe-OMe und Reaktion mit POA-His-OH] in 10 ml Methanol, hydriert an 0,5 g 10 %ig. Pd-C bei 20° und 1 bar 3 Std. lang, filtriert, dampft ein und erhält POA-His-DAPP-Ile-Phe-OMe, F. 113—115°. (Diese Verbindung wird nicht beansprucht!).

Analog erhält man durch Hydrogenolyse der entsprechenden CBZ-Derivate:

Benzoyl-His-DAMH-Ile-DAMH-OMe
Phenylacetyl-His-DAMH-Ile-DAMH-OMe
Phenoxyacetyl-His-DAMH-Ile-DAMH-OMe
3-Phenylpropionyl-His-DAMH-Ile-DAMH-OMe
2-Benzyl-3-phenylpropionyl-His-DAMH-Ile-DAMH-OMe
BOC-Phe-Nle-DACP-Ile-DAMH-OMe, F. 191° (Zers.).

BOC-Phe-Abu-DAMH-Ile-DAMH-OMe
BOC-Phe-Nle-DAMH-Ile-DAMH-OMe, F. 183—184°
BOC-Tic-His-DAMH-Ile-DAMH-OMe
Boc-(N-Me-Phe)-His-DAMH-Ile-DAMH-OMe
BOC-Phe-Orn-DAMH-Ile-DAMH-OMe
BOC-Phe-Lys-DAMH-Ile-DAMH-OMe
BOC-Phe-Dab-DAMH-Ile-DAMH-OMe
BOC-(N-Me-His)-His-DAMH-Ile-DAMH-OMe.

Beispiel 2

Analog Beispiel 1 erhält man aus 3S - CBZ - amino - 4S - [(3S - CBZ - amino - 4S - (BOC - Phe - His - amino) - 6 - methyl - heptanoyl) - Ile - amino] - 6 - methylheptansäuremethylester [F. 204—206°; erhältlich aus 3S - CBZ - amino - 4S - amino - 6 - methylheptansäuremethylester (Hydrochlorid, F. 148—149°) über 3S - CBZ - amino - 4S - (BOC - Ile - amino) - 6 - methylheptansäuremethylester, 3S - CBZ - amino - 4S - (Ile - amino) - 6 - methylheptansäuremethylester, 3S - CBZ - amino - 4S - (3S - CBZ - amino - 4S - BOC - amino - 6 - methylheptanoyl - Ile - amino) - 6 - methylheptansäuremethylester und 3S - CBZ - amino - 4S - (3S - CBZ - amino - 4S - amino - 6 - methylheptanoyl - Ile - amino) - 6 - methylheptansäuremethylester] durch Hydrogenolyse 3S - Amino - 4S - [(3S - amino - 4S - (BOC - Phe - His - amino) - 6 - methylheptanoyl) - Ile - amino] - 6 - methylheptansäuremethylester ("BOC-Phe-His-DAMH-Ile-DAMH-OMe"), F. 158—159° (Zers.).

**Patentansprüche**

1. Diaminosäurederivate der Formel I

$$X—Z—NH—CH(CH_2R^1)—CH(NH_2)—CH_2—CO—E—G—Y \qquad I$$

worin

- X — H, $R^2—O—C_nH_{2n}—CO—$, $R^2—C_nH_{2n}—O—CO—$, $R^2—C_nH_{2n}—CO—$, $R^2—SO_2—$, $(R^2—C_nH_{2n})—L(R^2—C_rH_{2r})—C_tH_{2t}—CO—$, $H—(NHCH_2CH_2)_n—NH—CH_2CO—$ oder 9-Fluorenyl-$C_nH_{2n}—O—CO—$,
- Z — 0 bis 4 peptidartig miteinander verbundene Aminosäurereste ausgewählt aus der Gruppe bestehend aus Abu, Ada, Ala, Arg, Dab, Gly, His, Ile, Leu, tert.-Leu, Lys, Met, Nbg, Nle, N-Me-His, N-Me-Phe, Orn, Phe, Pro, Ser, Thr, Tic, Trp, Tyr und Val,
- $R^1$ — H, A, Cycloalkyl mit 3—7 C-Atomen, Ar oder $C_pH_{2p}—W$,
- E — abwesend, Ala, Gly, Ile, Leu, tert.-Leu, Met, Ser, Thr oder Val,
- G — $—NH—CH(CH_2R^1)—CH(NH_2)—CH_2—CO—$,
- Y — $—O—C_mH_{2m}—R^3$, $—NH—C_mH_{2m}—R^3$, $—NH—C_mH_{2m-1}(R^3)_2$ oder $NA_2$,
- $R^2$ — A, Cycloalkyl mit 3—7 C-Atomen, Benzyl oder Ar,
- L — CH oder N,
- $R^3$ — H, A, Cycloalkyl mit 3—7 C-Atomen, Ar, Pyridyl, Imidazolyl, Piperidyl, N-Benzyl-piperidyl oder Piperazinyl,
- W — OH, $NH_2$, OA, NHA oder $NA_2$,
- A — Alkyl mit 1—6 C-Atomen,
- Ar — unsubstituiertes oder ein- oder mehrfach durch A, AO, F, Cl, Br, J, $CF_3$ und/oder $NH_2$ substituiertes Phenyl oder unsubstituiertes Naphthyl und

m, n, p, r und t jeweils 0, 1, 2, 3, 4 oder 5 bedeuten, sowie deren Salze.

2. Verfahren zur Herstellung eines Diaminosäurederivats der Formel I nach Patentanspruch 1 sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man ein Aminoketosäurederivat der Formel II

$$X—Z—NH—CH(CH_2R^1)—CO—CH_2—CO—E—G—Y \qquad II$$

worin

$R^1$, E, G, X, Y und Z die bei Formel I angegebene Bedeutung haben reduktiv aminiert und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine Verbindung der Formel I durch Behandeln mit einer Säure oder Base in eines ihrer Salze überführt.

3. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger- oder Hilfsstoff und gegebenenfalls in

Kombination mit einem oder mehreren weiteren Wirkstoff(en) in eine geeignete Dosierungsform bringt.

4. Pharmaceutische Zubereitung, gekennzeichnet durch einen Gehalt an mindestens einer Verbindung der Formel I nach Patentanspruch 1 und/oder einem ihrer physiologisch unbedenklichen Salze.

5. Verbindungen der Formel I nach Patentanspruch 1 und deren physiologisch unbedenkliche Salze zur Bekämpfung von Krankheiten.

6. Verwendung von Verbindungen der Formel I nach Patentanspruch 1 oder von deren physiologisch unbedenklichen Salzen zur Herstellung eines Arzneimittels mit der Indikation reninabhängige Hypertension oder Hyperaldosteronismus.

**Revendications**

1. Dérivés d'acides diaminés de formule I

$$X—Z—NH—CH(CH_2R^1)—CH(NH_2)—CH_2—CO—E—G—Y \quad I$$

dans laquelle

- X représente H, $R^2—O—C_nH_{2n}—CO—$, $R^2—C_nH_{2n}—O—CO$, $R^2—C_nH_{2n}—CO—$, $R^2—SO_2$, $(R^2—C_nH_{2n})—L(R^2—C_rH_{2r})—C_tH_{2t}—CO—$, $H—(NHCH_2CH_2)_n—NH—CH_2CO—$ ou 9-fluorényl-$C_nH_{2n}—O—CO—$,
- Z 0 à 4 restes d'acide aminé liés entre eux par liaison peptidique, choisis parmi le groupe comportant Abu, Ada, Ala, Arg, Dab, Gly, His, Ile, Leu, Leu tert., Lys, Met, Nbg, Nle, N-Me-His, N-Me-Phe, Orn, Phe, Pro, Ser, Thr, Tic, Trp, Tyr et Val,
- $R^1$ H, A, cycloalkyle avec 3 à 7 atomes de carbone, Ar ou $C_pH_{2p}—W$,
- E absent, Ala, Gly, Ile, Leu, Leu tert., Met, Ser, Thr ou Val,
- G $—NH—CH(CH_2R^2)—CH(NH_2)—CH_2—CO—$,
- Y $O—C_mH_{2m}—R^3$, $—NH—C_mH_{2m}—R^3$, $—NH—C_mH_{2m-1}(R^3)_2$ ou $NA_2$,
- $R^2$ A, cycloalkyle avec 3 à 7 atomes de carbone, benzyle ou Ar,
- L CH ou N,
- $R^3$ H, A, cycloalkyle avec 3 à 7 atomes de carbone, Ar, pyridyle, imidazolyle, pipéridyle, N-benzylpipéridyle ou pipérazinyle,
- W OH, $NH_2$, OA, NHA ou $NA_2$,
- A Alkyle avec 1 à 6 atomes de carbone,
- Ar phényle non substitué ou substitué de façon simple ou multiple par A, AO, F, Cl, Br, I, $CF_3$ et/ou $NH_2$, ou naphtyle non substitué, et

m, n, p, r et t signifient chaque fois 0, 1, 2, 3, 4 ou 5,

ainsi que leurs sels.

2. Procédé pour la fabrication d'un dérivé d'acide diaminé de formule I selon la revendication 1 ainsi que de ses sels, caractérisé en ce qu'on le libère d'un de ses dérivés fonctionnels par traitement avec un agent solvolysant ou hydrogénolysant ou qu'on amine par réduction un dérivé d'acide aminocétonique de formule II

$$X—Z—NH—CH(CH_2R^1)—CO—CH_2—CO—E—G—Y \quad II$$

dans laquelle

$R^1$, E, G, X, Y et Z ont les significations décrites pour la formule I et qu'on libère éventuellement dans un composé de formule I un groupe amino et/ou hydroxy fonctionnellement modifié par traitement avec un agent hydrogénolysant ou solvolysant et/ou qu'on transforme un composé de formule I en un de ses sels par traitement avec un acide ou une base.

3. Procédé pour la fabrication de préparations pharmaceutiques, caractérisé en ce qu'on met sous une forme de dosage appropriée un composé de formule I et/ou un de ses sels physiologiquement acceptables avec au moins un support ou adjuvant solide, liquide ou semi-liquide et éventuellement en combinaison avec une ou plusieurs autre(s) substance(s) active(s).

4. Préparation pharmaceutique caractérisée en ce qu'elle contient au moins un composé de formule I selon la revendication 1 et/ou un de ses sels physiologiquement acceptables.

5. Composés de formule I selon la revendication 1 et leurs sels physiologiquement acceptables pour la lutte contre les maladies.

6. Utilisation des composés de formule I selon la revendication 1 ou de leurs sels physiologiquement acceptables pour la fabrication d'un médicament indiqué pour le traitement de l'hyperaldostéronisme ou de l'hypertension en liaison avec la rénine.

**Claims**

1. Diamino acid derivatives of the formula I

$$X—Z—NH—CH(CH_2R^1)—CH(NH_2)—CH_2—CO—E—G—Y \quad I$$

wherein

X is H, $R^2—O—C_nH_{2n}—CO—$, $R^2—C_nH_{2n}—O—CO—$, $R^2—C_nH_{2n}—CO—$, $R^2—SO_2—$, $(R^2—C_nH_{2n})—L(R^2—C_rH_{2r})—C_tH_{2t}—CO—$, $H—(NHCH_2—CH_2)_n—NH—CH_2CO—$ or 9-fluorenyl-$C_nH_{2n}—O—CO—$,

Z is 0 to 4 amino acid radicals, bonded to one another in a peptide-like manner, selected from the group consisting of Abu, Ada, Ala, Arg, Dab, Gly, His, Ile, Leu, tert.-Leu, Lys, Met, Nbg, Nle, N-Me-His, N-Me-Phe, Orn, Phe, Pro, Ser, Thr, Tic, Trp, Tyr and Val,

$R^1$ is H, A, cycloalkyl with 3—7 C atoms, Ar or $C_pH_{2p}—W$,

E is absent or is Ala, Gly, Ile, Leu, tert.-Leu, Met, Ser, Thr or Val,

G is $—NH—CH(CH_2R^1)—CH(NH_2)—CH_2—CO—$,

Y $—O—C_mH_{2m}—R^3$, $—NH—C_mH_{2m}—R^3$, $—NH—C_mH_{2m-1}(R^3)_2$ or $NA_2$,

$R^2$ is A, cycloalkyl with 3—7 C atoms, benzyl or Ar,

L is CH or N.

$R^3$ is H, A, cycloalkyl with 3—7 C atoms, Ar, pyridyl, imidazolyl, piperidyl, N-benzyl-piperidyl or piperazinyl,

W is OH, $NH_2$, OA, NHA or $NA_2$,

A is alkyl with 1—6 C atoms,

Ar is unsubstituted phenyl, phenyl which is mono- or poly-substituted by A, AO, F, Cl, Br, I, $CF_3$ and/or $NH_2$, or unsubstituted naphthyl and

m, n, p, r and t are each 0, 1, 2, 3, 4 or 5,

and salts thereof.

2. Process for the preparation of a diamino acid derivative of the formula I according to Patent Claim 1 and of its salts, characterized in that it is liberated from one of its functional derivatives by treatment with a solvolysing or hydrogenolysing agent, or in that an amino-keto acid derivative of the formula II

$$X—Z—NH—CH(CH_2R^1)—CO—CH_2—CO—E—G—Y \quad II$$

wherein $R^1$, E, G, X, Y and Z have the meaning given in the case of formula I, is subjected to reductive amination, and in that, if appropriate, a functionally modified amino and/or hydroxyl group in a compound of the formula I is liberated by treatment with solvolysing or hydrogenolysing agents, and/or a compound of the formula I is converted into one of its salts by treatment with an acid or base.

3. Process for the preparation of pharmaceutical formulations, characterized in that a compound of the formula I and/or one of its physiologically acceptable salts is brought into a suitable dosage form together with at least one solid, liquid or semi-liquid excipient or auxiliary and, if appropriate, in combination with one or more other active compound(s).

4. Pharmaceutical formulation, characterized in that it contains at least one compound of the formula I according to Patent Claim 1 and/or one of its physiologically acceptable salts.

5. Compounds of the formula I according to Patent Claim 1 and their physiologically acceptable salts for combating diseases.

6. Use of compounds of the formula I according to Patent Claim 1 or of their physiologically acceptable salts for the preparation of a medicament with the indication of renin-dependent hypertension or hyperaldosteronism.